# EUROPEAN PATENT APPLICATION

(11) **EP 1 124 134 A2**
(43) Date of publication of application: **16.08.2001**
(21) Application number: 01830078.0
(22) Date of filing: 08.02.2001
(51) Int. Cl.: G01N 33/36

(54) **Process an apparatus for measuring the count of yarns**

(30) Priority: 11.02.2000 IT MI000236
(71) Applicant: Zuccolini, Livio, 20089 Rozzano MI (IT)
(72) Inventor: Breggion, Adriano, 20010 Bareggio MI (IT)
(74) Representative: Pizzoli, Antonio

(57) **Abstract**

Process for measuring the count of yarns, characterized by comprising the following operative steps:
- measuring the permittivity between the plates (15, 15', 16, 16') of at least one measurement capacitor;
- inserting at least one yarn (5, 5') between said plates (15, 15', 16, 16');
- measuring the permittivity between said plates (15, 15', 16, 16') with the yarn (5, 5') inserted;
- calculating the permittivity variation between said plates (15, 15', 16, 16') due to the insertion of the yarn (5, 5');
- calculating the running speed of the yarn (5, 5');
- calculating the count of the yarn (5, 5') according to said permittivity variation and said running speed.

The present invention also relates to an apparatus with carries out said process, as well as a process for manufacturing this apparatus.

## Description

The present invention relates to a process for measuring the count of yarns, and in particular to a process which can be used in the textile industry for measuring the count of yarns with wool fibers and the like. The present invention also relates to an apparatus with carries out said process, as well as a process for manufacturing this apparatus.

A process for the continuous measurement of yarns is known, which is based upon an apparatus comprising a capacitive type sensor with is arranged downstream the lapper which mixes and draws more ribbons with parallel fibers for providing a ribbon with a controlled count. The output signal from said sensor allows to adjust by means of feedback the drawing ratio of the ribbon, thereby keeping its count constant. However, since the ribbon uninterruptedly runs in the same position between the two plates of the condenser of said sensor, due to the continuous variations of the ambient conditions only the ratio variation with respect to a reference value, for instance that of a standard ribbon, can be measured, not the absolute count value of the ribbon itself.

It is therefore an object of the present invention to provide for a process and an apparatus free from said drawbacks, i.e. a process and an apparatus which allow to obtain an absolute yarn count measurement, which cannot be negatively influenced by the ambient conditions of the measurement. Said object is achieved with a process and an apparatus, the main features of which are specified in the first and fourth claim respectively, while other features are specified in the remaining claims.

The process according to the present invention, by carrying out a cyclic and alternated measurement of the permittivity variation between the plates of a measurement capacitor, due to the presence or absence of the yarn to be measured, allows to determine in an absolute manner the density of the yarn itself and therefore also the relevant count, which is calculated according to the running speed of the yarn, i.e. to the oscillation frequency of the yarn from the inside to the outside of the measurement capacitor, and vice versa.

Furthermore, according to a particular advantageous aspect of the invention, at each cycle a plurality of measurements of the permittivity variations between two pairs of plates of two measurement capacitors is carried out, as well as a balanced zero setting of the permittivity measurements between said pairs of plates when the yarn is not inserted between them. With this arrangement, it is not only possible to contemporaneously measure the count of two yarns, but also the negative influence of the temperature and humidity of the ambient is neutralized, since the zero settings take place at very short intervals, in particular shorter than a second.

Thanks to the sensor which is provided with, the apparatus according to the present invention therefore acts as a two pan scale wherein, before each measurement, the zero value, i.e. the equilibrium between the two pans, is precisely adjusted. This apparatus is particularly useful if employed for measuring the count of the rovings coming from the finishing machines, such as for instance the machines for inserting a roving twist. Now, these rovings can be measured only through periodic controls of samples in a laboratory, since the known apparatuses do not provide for a suitable measurement precision.

At last, thanks to the particular process for manufacturing the sensors of the apparatus according to the present invention, it is possible to arrange at a same mutual distance more pairs of plates in a single sensor, so as to improve the measurement precision of the apparatus itself. As a matter of fact, practical tests show that the apparatus according to the present invention allows to obtain measurements with errors only of about 0,15%, in comparison with measurements with errors greater than 2% carried out by means of the above mentioned known apparatuses, with the same kind of yarns.

Further advantages and features of the process and apparatus according to the present invention will be clear to those skilled in the art from the following detailed and non-limiting description of an embodiment thereof, with reference to the attached drawings, wherein:
- figure 1 shows a front view of the apparatus according to the present invention applied close to two collecting bobbins of a finishing machine;
- figure 2 shows a bottom view in a cross-section of a sensor of the apparatus of figure 1;
- figure 3 shows a block diagram of the sensor of figure 2;
- figure 4 shows a block diagram of a secondary electronic device of the apparatus of figure 1.

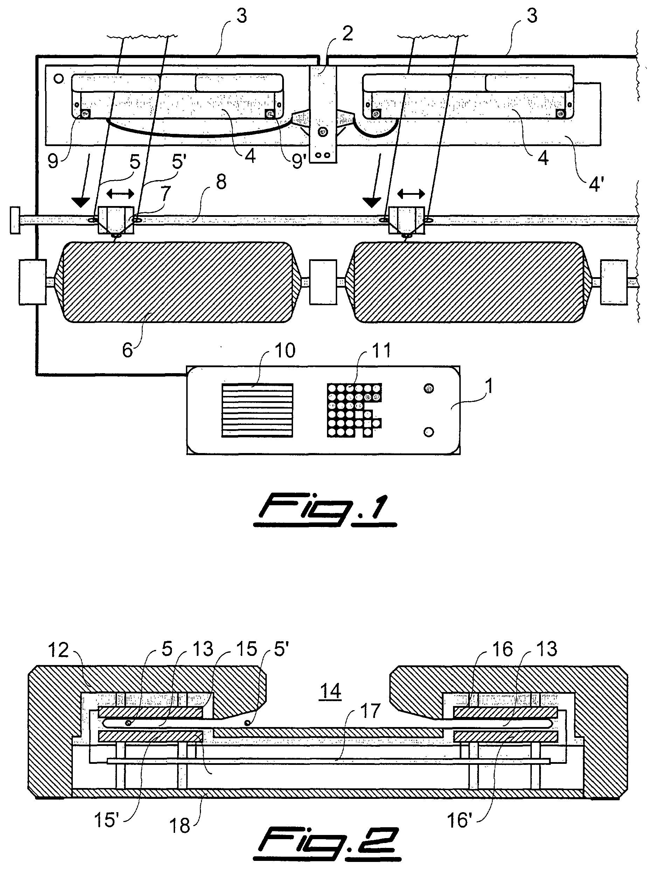

Referring to figure 1, it is seen that the apparatus according to the present invention comprises an electronic control unit 1 connected in series to one or more secondary electronic devices 2 through a plurality of data cables 3. One or more sensors 4 of the capacitive type (two are shown in the figure), each arranged onto a fixing plate 4' and measuring the count of a pair of yarns 5, 5', for instance two rovings in a finishing machine, are in turn connected to each of these secondary devices 2.

Said yarn pair 5, 5' is homogeneously wound around a bobbin 6, for instance the collecting bobbin of said finishing machine, by means of a pair of mobile thread guides 7 which move with an alternated motion along a guide 8 arranged parallel to the axis of bobbin 6. Plate 4' of sensors 4 is suitably fixed to the finishing machine close to said mobile thread guides 7 so that yarns 5, 5' can move in these sensors with the same alternated motion. Two pilot lamps 9, 9' suitable for immediately signaling to the users a defect, in any, detected in one of the two yarns 5, 5' which cross the sensors 4, are further arranged close to both lateral edges of sensors themselves. Control unit 1 is instead provided with a display 10 suitable for showing the data detected by the different sensors 4 connected thereto, as well as a keyboard 11 which allows the user to control the working of the apparatus according to the present invention.

With reference to figure 2, it is seen that said sensors 4 comprise a hollow body 12, for instance made of aluminum, crossed by at least a transversal slit 13 in which the yarn pair 5, 5' can run and move with an alternated motion. In the present embodiment slit 13 is open to the outside through a central cavity 14. Two pairs of metal members 15, 15' and 16, 16' acting as plates of two measurement capacitors, each arranged respectively close to the left and right ends of slit 13, are in turn arranged astride the two largest opposite surfaces of slit 13. Each plate 15, 15', 16 and 16' is electrically connected to a board 17 comprising a plurality of electric and electronic devices mutually connected by means of an electric circuit, for instance a printed board. Board 17 is accessible from the outside through a removable cover 18 arranged on one of the largest sides of the hollow body 12.

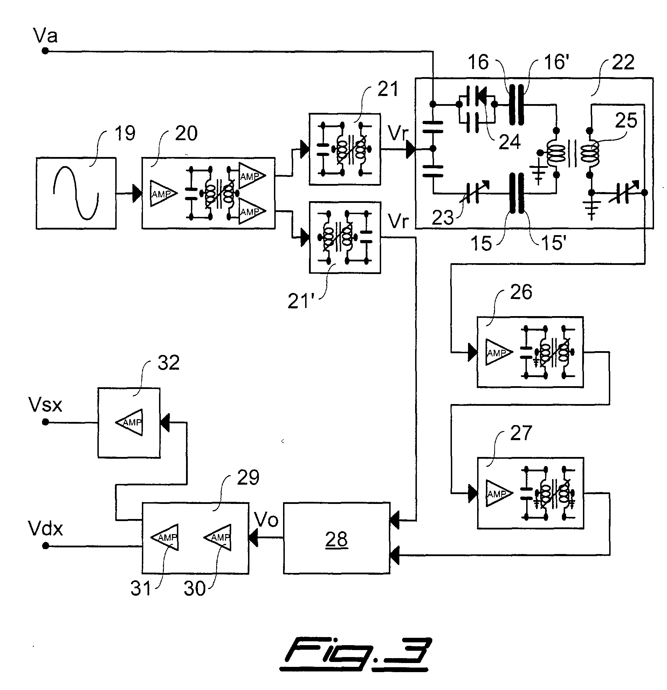

Referring now to figure 3, it is seen that said electric and electronic devices arranged into sensors 4 comprise an oscillating circuit 19 which provides for an electric signal with a determined frequency, for instance a sinusoidal wave at 10,7 MHz, to a resonating circuit 20 comprising one input and two output amplification stages, connected in parallel.

The two sinusoidal signals transmitted by the outputs of the resonating circuit 20 are in turn connected to two further resonating circuits 21, 21', each outputting a reference signal Vr, the voltage of which has constant amplitude Vpp and phase φ. One of said signals is transmitted to a measurement circuit 22 wherein the signal amount sent to plates 15, 15' and 16, 16' of the left and right measurement capacitors is shared out. Said sharing provides for a rough adjustment by means of a variable capacitor 23 connected in series to plate 15 and a fine adjustment through a zero setting signal Va coming from a secondary device 2 and transmitted to a varicap diode 24 connected in series to plate 16.

By means of these adjustments, when the dielectric between plates 15, 15' and 16, 16' is made of air only, two currents having the same intensity are obtained in the primary circuit of a bobbin 25 with the central point connected to earth, which is connected downstream the measurement capacitors. When two equal vector values, but always in a phase opposition, are reached, the current in the secondary circuit of the bobbin will be zero. The variation, if any, of the dielectric in only one of the two measurement capacitors, in particular due to the presence of yarns 5 or 5', generates at the output of the measurement circuit 22 a sinusoidal signal with a voltage having an amplitude directly proportional to the permittivity variation of one capacitor, but with a coincidence or opposition of phase φ according to the permittivity variation of the left or right capacitor, respectively.

The output signal from measurement circuit 22 passes through a resonating circuit 26 comprising an input amplification stage and arrives to a resonating circuit 27 comprising a video amplification stage, which introduces a 45° phase difference in the signal.

The output signals from resonating circuits 21' and 27 come to a phase comparing circuit 28 which outputs a signal Vo, the voltage of which has an amplitude proportional to the permittivity variation and a positive or negative sign according to the comparison carried out between the phase of the reference signal Vr coming from circuit 21' and the output signal of circuit 27.

An integration circuit 29 which eliminates the residual voltage Vpp from signal Vo is arranged downstream the phase comparing circuit 28. Circuit 29 comprises an amplification and integration stage 30, as well as an amplifier 31 with gain regulation which allows the calibration of the positive and negative values of signal Vo according to the permittivity variation in the measurement capacitors with a calibration template simulating a standard yarn having a determined count. Circuit 29 therefore outputs a signal Vdx proportional to the permittivity variation between plates 16, 16' in the right measurement capacitor. Signal Vdx is also transmitted to an inverter circuit 32 with an unitary gain, so as to obtain a second signal Vsx proportional to the permittivity variation between plates 15, 15' in the left measurement capacitor. By operating on the inverter circuit 32 it is possible to obtain a slight correction of the gain for compensating deviations, if any, and therefore making the positive value of signal Vsx equal to the previous positive value Vdx measured with the same calibration template.

Since in the textile industry the finishing machines produce rovings with a variable count comprised between 0,2 and 1,6 g/m, the distance between plates 15, 15' and 16, 16' can vary, so as to obtain the maximum sensibility and measurement precision for the whole range of possible counts. In particular, the distance between plates 15, 15' and 16, 16' is 3 mm for counts from 0,2 to 0,8 g/m, and 4 mm for counts from 0,4 to 1,6 g/m. The indication of this distance to the apparatus is carried out by inserting of removing an electric jumper (non visible in the figure).

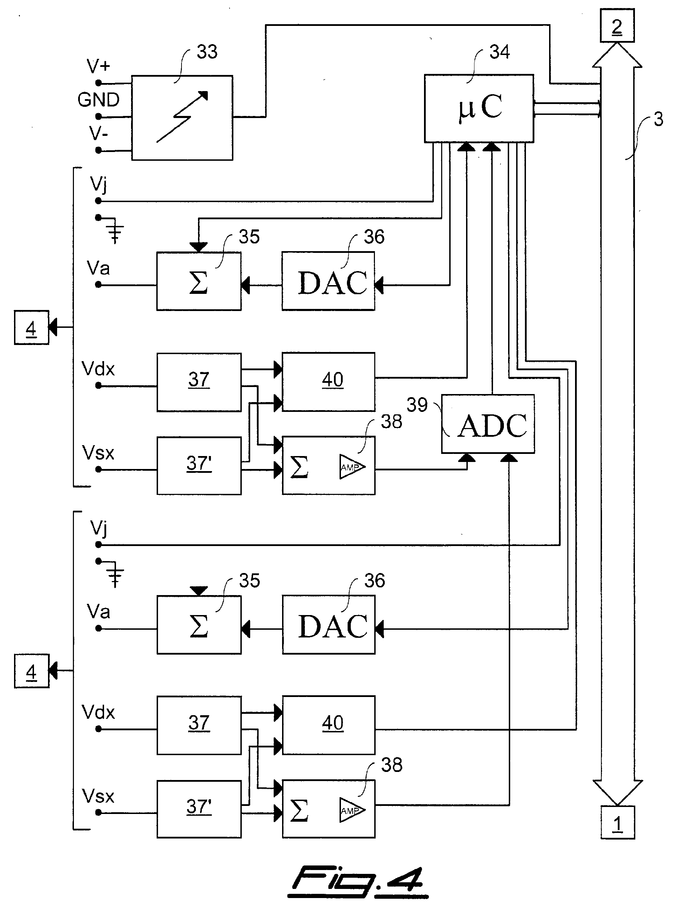

With reference to figure 4, it is seen that a secondary electronic device 2 comprises an aluminum container in which three boards are arranged one above the other. The first board comprises a power circuit 33 which supplies electric power with stabilized voltages V+ and V-, for instance +5 V, +12 V and -12 V, to boards 17 of sensors 4 connected to the secondary device 2 and to the two remaining boards of the device itself, which comprise electric circuits for controlling sensors 4.

Signals Vj depending upon the position of said electric jumper are instead transmitted to a digital microcontroller 34, in particular to a 16-bit microcontroller provided with a CAN interface. Microcontroller 34 transmits a pulse width modulation (PWM) signal to the boards for sensors 4, which comprise a pair of summation circuits 35, each summing said input signal with a further signal coming from a digital-analog converter 36, in particular at 12 bit, in turn connected to microcontroller 34. In each summation circuit 35 the values of the pulse modulation width signal, acting as a rough adjustment, and of the signal coming from the relevant converter 36, acting as a fine adjustment, are summed with a reference voltage of 5 V, so as to obtain a voltage Va comprised between 5 and 7,5 V, devoted to the circuits of sensors 4.

The analog signal Vsx and Vdx coming from sensors 4 are respectively inputted to a pair of filtering circuits 37, 37' which eliminate the negative portion of the signal themselves. Circuits 37, 37' are connected to a further summation circuit 38, provided with an output amplification stage, which collects signals Vsx and Vdx in a single signal. The latter is transmitted to an analog-digital converter 39, in particular at 12 bit, which is connected to microcontroller 34. The signal outputted by filtering circuits 37, 37' are also sent to an analog-digital detecting circuit 40 which is connected to microcontroller 34 for detecting the exceeding a determined threshold value Vt, for instance about 200 mV, so as to determinate the presence or absence of yarns 5, 5' in the relevant measurement capacitors. Through the output signal from detecting circuit 40 it is also possible to control whether it is necessary to increase or decrease the value of voltage Va for obtaining a perfectly zero value.

The data transmitted in a digital form from detecting circuit 40 to microcontroller 34 are processed by the latter also for controlling the sampling frequency of the analog-digital converter 39, so as to automatically adapt the apparatus according to the present invention to the production speed of the finishing machine, i.e. to the running speed of yarns 5, 5', which is a variable defined by the users. With this arrangement it is possible to carry out inside a measurement capacitor a number of instantaneous measurements of the yarn count which is equal to or greater than a determinate minimum value, for instance equal to 100, whichever the permanence time of yarns 5, 5' between the capacitors is. Said permanence time obviously depends upon the number of oscillation of yarns 5, 5' in slit 13, which is generally comprised between 20 and 300 per minute. The subsequent processing of microcontroller 34 outputs only two values Vsx and Vdx per oscillation, which are an arithmetical mean of the single instantaneous measurements.

Signaling lights and switches for activating and signaling the working of each sensor 4 are then arranged onto the external portion of the secondary devices 2.

The above mentioned signals are transmitted and received between the control unit 1 and all the secondary devices 2 of the apparatus according to the present invention through a CAN protocol, while through a data network, for instance of the Ethernet type, the control units 1 of more apparatuses according to the present invention can be controlled by a central computer (not shown in the figures).

Control unit 1 includes a computer of the known type having at least a microprocessor and a digital memory for executing a program that sends to the different microcontrollers 34 of the secondary devices 2 the setup data and working parameters defined by the users, and receives therefrom the data of the measurements and of the local processing.

In control unit 1 the users have at their disposal more separate data channels for the measurement of the yarn counts and can program for each channel some threshold values of the count variation according to the number of oscillations, i.e. lengths, of the yarns themselves. If said threshold values are exceeded, pre-alarm or alarm states can be activated, so as to stop the production, if necessary.

In particular, a first channel can be set for detecting defects of the yarns in a short term, for instance with threshold values of -5 % and 6 % for a number of oscillations equal to or greater than 2. A second channel can be instead set for detecting defects of the yarns in a middle term, for instance with threshold values of -1,6 % and 1,8 % for a number of oscillations equal to or greater than 34. A third channel can then be set for detecting defects of the yarns in a long term, for instance with threshold values of -1,4 % and 1,4 % for a number of oscillations equal to or greater than 1000. The latter channel is not referred to single yarns, as it is with the first two channels, but is a weighted mean of all the values of the single yarns.

During the use, yarns 5, 5' oscillate with an alternated motion in slit 13 between plates 15, 15' of the left measurement capacitor and plates 16, 16' of the right measurement capacitor. When both yarns 5, 5' are in the middle of slit 13, the permittivity of the capacitors is equal to the dielectric constant of the air. The negative influence of the temperature and of the humidity of the ambient is neutralized by forcing a balanced zero setting of the electric output signals Vsx and Vdx. When both yarns 5, 5' instead move toward an end of slit 13, only one of the two yarns comes between the plates of a measurement capacitor, so as to carry out a series of measurements on said yarn. At last, the mean of the measurements carried out serves for obtaining in an univocal manner the count of yarns 5, 5' according to the kind of measured fiber.

For manufacturing the above mentioned single electric and electronic devices it is possible to employ techniques and technologies known for those skilled in the art, while for the manufacturing of sensors 4 the hollow body 12 is suitably manufactured in a first step without slit 13 and cavity 14. In a second operative step two shaped copper plates, each provided with two reophores, are fixed into the hollow body 12 by means of a epoxy resin bath with a high dielectric strength. In the third step, when the resin has hardened, slit 13 and cavity 14 are carried out in the hollow body 12, so as to cut in two portions said copper plates and obtain the pair of plates 15, 15' and 16, 16' having an identical mutual distance.

## Claims

1. Process for measuring the count of yarns, characterized by comprising the following operative steps:
- measuring the permittivity between the plates (15, 15', 16, 16') of at least one measurement capacitor;
- inserting at least one yarn (5, 5') between said plates (15, 15', 16, 16');
- measuring the permittivity between said plates (15, 15', 16, 16') with the yarn (5, 5') inserted;
- calculating the permittivity variation between said plates (15, 15', 16, 16') due to the insertion of the yarn (5, 5');
- calculating the running speed of the yarn (5, 5');
- calculating the count of the yarn (5, 5') according to said permittivity variation and said running speed.

2. Process according to the previous claim, characterized in that said operative steps are cyclically repeated.

3. Process according to the previous claim, characterized in that at each cycle a plurality of measurements of the permittivity variations between two pairs of plates (15, 15', 16, 16') of two measurement capacitors is carried out, as well as a balanced zero setting of the permittivity measurements between said pairs of plates (15, 15', 16, 16') when the yarn (5, 5') is not inserted between them.

4. Apparatus for measuring the count of yarns, which comprises at least one sensor of the capacitive type, characterized in that said sensor (4) includes at least one slit (13) in which one or more yarns (5, 5') can oscillate, as well as one or more measurement capacitors, each provided with a pair of plates (15, 15', 16, 16') which are arranged astride the slit (13) and are connected to at least one electric and/or electronic circuit (17) suitable for measuring the permittivity of the measurement capacitors during the oscillation of the yarns (5, 5') in the slit (13).

5. Apparatus according to the previous claim, characterized in that said sensor (4) comprises at least two measurement capacitors arranged close to the two ends of the slit (13).

6. Apparatus according to claim 4 or 5, characterized in that it comprises means (4') for fixing one or more sensors (4) close to one or more mobile thread guides (7) which move with an alternated motion in a machine for finishing yarns (5, 5').

7. Apparatus according to claim 5 or 6, characterized in that the pairs of plates (15, 15', 16, 16') of the measurement capacitors are comprised in a measurement circuit (22) which inputs an alternated reference electric signal (Vr) with a voltage having constant amplitude (Vpp) and phase (φ) and outputs an alternated signal with an amplitude directly proportional to the permittivity variation of one capacitor, but with a coincidence or opposition of phase (φ) according to the permittivity variation of the first or second measurement capacitor, respectively.

8. Apparatus according to the previous claim, characterized in that said measurement circuit (22) comprises adjustment means (23, 24) for sharing out the amount of the reference alternated electric signal (Vr) sent to the pair of plates (15, 15', 16, 16') of the two measurement capacitors.

9. Apparatus according to one of claims 5 to 8, characterized in that it comprises an integration circuit (29) suitable for outputting a first electric signal (Vdx) proportional to the permittivity variation between the plates (16, 16') of the first measurement capacitor, said signal being transmitted to an inverter circuit (32) with an unitary gain which outputs a second electric signal (Vsx) proportional to the permittivity variation between the plates (15, 15') of the second measurement capacitor.

10. Apparatus according to the previous claim, characterized in that it comprises digital circuits (34, 40) for detecting the oscillation frequency of the yarns (5, 5') in the slit (13) of the sensors (4).

11. Apparatus according to the previous claim, characterized in that it comprises digital circuits (39) for sampling said electric signals (Vsx, Vdx) proportional to the permittivity variation between the plates (15, 15', 16, 16') of the measurement capacitors, wherein the sampling frequency of these sampling circuits (39) is proportional to the frequency of the oscillations of the yarns (5, 5') in the slit (13) of the sensors (4).

12. Apparatus according to the previous claim, characterized in that it comprises digital circuits (34) for carrying out an arithmetical mean of the electric signals (Vsx, Vdx) sampled by means of said sampling circuits (39).

13. Apparatus according to one of claims 4 to 12, characterized in that it comprises at least one electronic control unit (1) provided with a display (10), a keyboard (11), a microprocessor and a digital memory, wherein said control unit (1) is connected through a plurality of data cables (3) to one or more secondary electronic devices (2), each connected in turn to one or more capacitive sensors (4).

14. Apparatus according to the previous claim, characterized in that said control unit (1) is provided for the measurement of the count of the yarns (5, 5') with a plurality of data channels, each suitable for programming some threshold values of the count variation according to the number of oscillations of the yarns themselves.

15. Apparatus according to one of claims 4 to 14, characterized in that the distance between the plates (15, 15', 16, 16') of the measurement capacitors is comprised between 3 and 4 mm.

16. Process for manufacturing at least one sensor (4) of the apparatus according to one of claims 5 to 15, characterized in that it comprises the following operative steps:
- inserting at least two metal plates, each provided with two reophores, in a hollow body (12);
- fixing said metal plates to the hollow body (12) by means of a epoxy resin bath;
- carrying out the slit (13) of the sensor (4) in the hollow body (12), so as to cut in two portions said metal plates and obtaining said pairs of plates (15, 15' and 16, 16') of the two measurement capacitors.
